# NEUE EUROPÄISCHE PATENTSCHRIFT

(11) **EP 1 837 029 B2**
(45) Veröffentlichungstag und Bekanntmachung des Hinweises auf die Entscheidung über den Einspruch: **21.09.2016**
(45) Hinweis auf die Patenterteilung: 08.10.2008
(21) Anmeldenummer: 06006149.6
(22) Anmeldetag: 24.03.2006
(51) Int. Cl.: A61K 36/185

(54) **Zusammensetzung zur Vorbeugung und Behandlung von Erkältungskrankheiten**
Composition for prevention and treatment of coughs and colds
Composition pour la prévention et le traitement des coups de froid.

(43) Veröffentlichungstag der Anmeldung: 26.09.2007
(73) Patentinhaber: Pandalis, Georgios, 49219 Glandorf (DE)
(72) Erfinder: Pandalis, Georgios, 49219 Glandorf (DE)
(74) Vertreter: Grünecker Patent- und Rechtsanwälte PartG mbB

(56) Entgegenhaltungen:
- DE-U-202005 014 459
- DE-U-202005 014 460
- BLANK I: "Phytopharmaceuticals: Cistus incanus extract relieves sore throat" DEUTSCHE APOTHEKER ZEITUNG 17 NOV 2005 GERMANY, Bd. 145, Nr. 46, 17. November 2005 (2005-11-17), Seiten 40-41, XP001247104 ISSN: 0011-9857
- DATABASE WPI Section Ch, Week 200039 Derwent Publications Ltd., London, GB; Class B04, AN 2000-449914 XP002381134 & RU 2 138 279 C1 (SOLAGRAN LTD) 27. September 1999 (1999-09-27)
- VERYKOKIDOU E ET AL: "ANTIBACTERIOPHAGE PROPERTIES OF SOME GREEK PLANT EXTRACTS" 1995, INTERNATIONAL JOURNAL OF PHARMACOGNOSY, SWETS & ZEITLINGER, LISSE, NL, PAGE(S) 339-343 , ATHENS, GREECE , XP008060533 ISSN: 0925-1618 * das ganze Dokument *

## Beschreibung

Die vorliegende Erfindung betrifft die Verwendung von Cistus zur Herstellung einer Zusammensetzung bzw. eines Präparats zur Prophylaxe von Erkältungskrankheiten (grippalen Infekten), *die eine Primärinfektion, hervorgerufen durch Rhinoviren, umfassen.*

Zu den typischen Erkältungskrankheiten zählen Atemwegsinfekte, wie Schnupfen und Mandel- und Rachenentzündungen sowie Husten und Bronchitis. Meist treten sie nacheinander auf; die Erkältung kann aber auch auf Nase, Hals oder Bronchien beschränkt bleiben. Derartige Erkältungskrankheiten werden auch als "grippaler Infekt" bezeichnet. Davon zu unterscheiden ist eine durch Influenza-Viren ausgelöste "echte" Grippe, die sogenannte Influenza, die einen deutlich längeren und schwereren Krankheitsverlauf aufweist und in der Regel mit Fieber verbunden ist.

Auch die genannten Erkältungskrankheiten werden durch Viren ausgelöst. Da es beispielsweise mehr als hundert verschiedene Typen von Viren gibt, die einen Schnupfen verursachen können, wird es kaum je möglich sein, einen Impfstoff dagegen zu entwickeln. Eine Behandlung von Schnupfen oder von Erkältungen allgemein ist daher auf eine Linderung der Symptome gerichtet. Meist werden hierbei altbewährte Hausmittel eingesetzt. Beispielsweise hilft bei stark verstopften Nasen das Inhalieren von heißem Dampf. Es lässt die Nasenschleimhaut abschwellen und fördert den Schleimausfluss. Unterstützend wirkt z. B. das Zufügen von einigen Tropfen Teebaum- oder Kamillenöl in das heiße Wasser. Es ist auch bekannt, dass ein regelmäßiges Durchspülen der Nase mit einer Salzlösung die Anfälligkeit für Schnupfen senken kann.

Neben den Selbsthilfemaßnahmen können Medikamente helfen, die Gefäße in der geschwollenen Nasenschleimhaut zu verengen, was zu einer Beruhigung der Nasenschleimhaut führt. Nasentropfen zur Abschwellung der Nasenschleimhaut sollten jedoch nicht länger als zwei bis drei Tage angewendet werden. Danach kann es sein, dass nach dem Absetzen die Nasenschleimhaut um so stärker anschwillt und sich ein "Medikamentenschnupfen" (Rhinitis medicamentosea) entwickelt.

Urheimische Notizen, Ausgabe 4/2003, beschreibt, dass für einen Schnupfen vor allem Rhinoviren, die in die Nasenschleimhäute eindringen, verantwortlich sind. Wenn daraufhin die Nase verstopft ist, helfen Nasentropfen auf Cystus^{®} Basis, da die darin enthaltenen Polyphenole abschwellend und entzündungshemmend wirken.

G. Harnisch, Cystus, Turm Verlag 2000, ISBN 3-7999-0265-1, beschreibt die griechische Wildpflanze Cystus.

Phytopharmaka sind im Gegensatz zu chemisch-synthetischen Nasensprays nebenwirkungsarm. Auch wenn sie über einen längeren Zeitraum eingenommen werden, schädigen sie die Nasenschleimhaut nicht und führen zu keiner Rhinitis medicamentosea. Je früher Phytopharmaka angewandt werden, desto wirksamer sind sie. Schon bei den ersten Anzeichen einer Erkältung können sie unterstützend eingesetzt werden. Sie wirken auch einer Ausbreitung der Infektion entgegen.

Beispielsweise werden häufig Echinacea-Präparate bei Erkältungskrankheiten eingenommen, wobei eine Vielzahl von unterschiedlichen Medikamenten in variabler phytochemischer Zusammensetzung auf dem Markt ist. Kontrollierte Studien über die Wirksamkeit dieser Phytotherapeutika existieren jedoch nur in begrenztem Umfang und mit widersprüchlichen Ergebnissen. Erst kürzlich hat sich jedoch in einer neuen Studie ergeben, dass Echinacea nicht die postulierte Wirksamkeit aufweist. Die Studie wurde mit drei Echinacea-Präparationen mit unterschiedlichem phytochemischen Profil, die durch Extraktion von E.-angustifolia-Wurzeln mit Kohlendioxid, 60% Ethanol oder 20% Ethanol gewonnen worden waren, durchgeführt. Insgesamt nahmen 437 Freiwillige mit Rhinovirus-Infektion an dieser Studie teil, die das Medikament entweder als Prophylaxe sieben Tage vor Virusexposition erhielten oder zur Behandlung zum Zeitpunkt der Exposition. Die Studie wurde placebokontrolliert durchgeführt. Zwischen den drei Echinacea-Extrakten und dem Placebo ergab sich kein signifikanter Unterschied bezüglich Infektionsrate, Schwere der Symptome, Volumen der Nasensekretion, Leukozytenzahl, Interleukin-8-Konzentration im Nasenspülwasser oder quantitativen Virustitern (Deutsches Ärzteblatt 102, Ausgabe 48 vom 02.12.2005, Seite A-3341 / B-2822 / C-2640 und New England Journal of Medicine, 2005, 353, 341-348).

Ein weiteres Medikament auf pflanzlicher Basis ist ein Extrakt aus den Wurzeln von Pelargonium *reniforme* oder *sidoides,* das unter dem Namen Umckaloabo^{®} vertrieben wird. Umckaloabo^{®} findet traditionell nicht nur bei Atemwegserkrankungen, sondern auch bei gastrointestinalen Erkrankungen Anwendung. Als wirksamkeitsbestimmende Inhaltsstoffe gelten zur Zeit eine Reihe von antibakteriellen und immunmodulierenden Inhaltsstoffen, wie Cumarine und Gerbstoffe. Es wird postuliert, dass der Extrakt antibakterielle, antivirale und sekretolytische Wirkungen entfaltet, wobei das Medikament von Schwangeren und Stillenden sowie bei Patienten mit Leber- und Nierenerkrankungen oder erhöhter Blutsneigung nicht angewendet werden soll, da in diesem Bereich noch nicht ausreichend Erfahrungen gesammelt werden konnten. Verglichen mit anderen Phytopharmaka ist Umckaloabo^{®} zudem recht kostspielig.

Aufgabe der vorliegenden Erfindung ist es deshalb, eine antivirale Zusammensetzung zur Prophylaxe von Erkältungskrankheiten (grippalen Infekten), *die eine Primärinfektion, hervorgerufen durch Rhinoviren, umfassen,* zur Verfügung zu stellen, die kostengünstig hergestellt werden kann und keinerlei oder nur geringe Nebenwirkungen bei der Verabreichung hervorruft.

Diese Aufgabe wird durch die Verwendung von Cistus zur Herstellung einer Zusammensetzung mit antiviraler Aktivität gegen Rhinoviren zur Hemmung der Infektiosität von Rhinoviren zur Prophylaxe von Erkältungskrankheiten (grippalen Infekten), *die eine Primärinfektion, hervorgerufen durch Rhinoviren, umfasst,* gelöst.

### Beschreibung der Figuren

Figur 1 zeigt die Ergebnisse eines Viabilitätstest mit Propidiumiodid-Färbung von A549-Zellen, die mit Cistus-Extrakt behandelt worden sind.
Figur 2 zeigt die Ergebnisse eines Viabilitätstest mit Propidiumiodid-Färbung von MDCK-Zellen, die mit Cistus-Extrakt behandelt worden sind.
Figur 3 zeigt die Ergebnisse eines Apoptose-Tests von A549-Zellen, die mit Cistus-Extrakt behandelt worden sind.

### Detaillierte Beschreibung der Erfindung

Als Erkältungskrankheiten im Sinne der Erfindung werden Entzündungen der Atemwege, also in der Regel von Nase, Rachen, Kehlkopf, Luftröhre und Bronchien, verstanden. Die Termini "Erkältungskrankheiten" und "grippaler Infekt" werden hierbei synonym gebraucht. Ein grippaler Infekt grenzt sich von einer "echten" Grippe bzw. Influenza dadurch ab, dass letztere nur durch Influenzaviren verursacht wird.

Ein grippaler Infekt wird dagegen meistens durch Adenoviren, Coronaviren und/oder Rhinoviren hervorgerufen.

Adenoviren (Adenoviridae) gehören zur Familieder kubischen DNA-Viren ohne Hüllmembran und weisen einen Durchmesser von 60 bis 90 nm auf. Das Genom besteht aus einer linearen doppelsträngigen DNA von ungefähr 36 kb Länge. Man unterscheidet ca. 50 immunologisch unterschiedliche Arten von Adenoviren, davon ca. 35 humanpathogene Typen in den Subgenera A-F. Die Familie der Adenoviridae wird in die Gattungen Mastadenoviren, die Säugetieren infizieren können, und Aviadenoviren, die in verschiedenen Vogelarten endemisch sind, eingeteilt. Adenoviren zeichnen sich durch eine ungewöhnliche Stabilität gegenüber chemischen und physikalischen Einwirkungen aus und tolerieren widrigste pH-Werte, was ihnen eine vergleichsweise lange Überlebenszeit außerhalb des Wirtskörpers ermöglicht.

Adenoviren verursachen hauptsächlich Erkrankungen der Atemwege. Abhängig vom jeweiligen Serotyp können allerdings auch eine Reihe anderer Erkrankungen hervorgerufen werden, so beispielsweise Gastroenteritis,. Konjunktivitis, Zystitis, Pharyngitis oder Durchfälle. Die Symptome der Atemwegserkrankung durch Adenoviren reichen von der einfachen Erkältung über die Bronchitis bis zur Pneumonie. Bei Patienten mit geschwächtem Immunsystem besteht eine besondere Anfälligkeit für ernsthafte Komplikationen der Adenoviren-Infektionen, wie zum Beispiel das ARDS (Acute Respiratory Distress Syndrome). Außerdem wird vermutet, dass es einen Zusammenhang zwischen dem Virustyp Ad-36 und der Fettleibigkeit beim Menschen gibt.

Coronaviren, welche zum Genus Coronaviridae gehören, verursachen beim Menschen in der Regel milde Erkrankungen der oberen Atemwege, selten Gastroenteritis und das Schwere Akute Respiratorische Syndrom (SARS), hervorgerufen durch das SARSassoziierte Coronavirus SARS-CoV.

Die Coronaviren werden zur Familie der pleomorphen RNA-Viren mit Hüllmembran und einem Durchmesser von 70 bis 160 nm gerechnet. Sie weisen eine Einzel(+)-Strang-RNA von 20 bis 30 kb Länge auf. Der Genus Coronaviridae wird in drei Genera unterteilt, die Coronaviren, die Arteriviren und die Toroviren. Von diesen umfassen lediglich die Coronaviren humanpathogene Viren. Die Übertragung der Viren erfolgt über Tröpfcheninfektion (aerogen), als Schmutz- und Schmierinfektion (fäkal-oral) oder auch durch einfachen Kontakt (mechanisch) mit einer infizierten Person. Jüngere infizierte Organismen können hierbei schwerer erkranken als ältere. Coronaviren verursachen zwischen 15 bis 30% der Erkältungskrankheiten des Menschen mit leichtem Fieber, Schnupfen, Husten und Halsschmerzen.

Als Rhinitis, Nasenkatarrh, Koryza oder umgangssprachlich Schnupfen wird eine akute oder chronische Reizung der Nasenschleimhaut mit den Symptomen Juckreiz, Niesreiz, Sekretion und Verstopfung durch infektiöse, allergische und nichtallergische Mechanismen bezeichnet. Der Erreger ist meist ein Genus des Picornavirus - das Rhinovirus. Die Infektion mit Rhinoviren erfolgt durch eine direkte Übertragung, z.B. über kontaminierte Hände oder auch über Tröpfcheninfektion.

Bisher sind über 115 Serotypen dieses Genus bekannt. Rhinoviren besitzen eine einsträngige (+)-RNA (messenger R NA) mit einer Länge von 7,2 bis 8,5 kb. Es handelt sich um nackte Viren mit einer icosahedralen Struktur und einem Durchmesser von 24 bis 30 nm. Die die RNA umgebende 10 bis 15 nm dicke Proteinhülle (Kapsid) besteht aus 60 symmetrisch angeordneten Untereinheiten, die als Protomere bezeichnet werden. Jedes Protomer besteht aus den vier Kapsidproteinen VP1, VP2, VP3 und VP4. Die Vielzahl der Protomere gilt als die Ursache für die Antigen-Vielfältigkeit der Rhinoviren.

Wie bereits erwähnt, werden Erkältungskrankheiten meist von Adenoviren, Coronaviren und/oder Rhinoviren hervorgerufen. Je nach Art des infektiösen Virus können Erkältungsbeschwerden, wie Schnupfen, Husten, Heiserkeit, Halsschmerzen, beispielsweise hervorgerufen durch Mandel- und Rachenentzündungen, Glieder- und Kopfschmerzen, Schüttelfrost, leichtes Fieber und Abgeschlagenheit, auftreten. Zu einer Erkältungserkrankung zählt auch eine Bronchitis und eine Bronchopneumonie.

Von diesen Erkältungskrankheiten tritt ein Schnupfen in den Wintermonaten am häufigsten auf. Er wird durch eine Infektion mit Rhinoviren, seltener auch mit Adenoviren, hervorgerufen. Die beschriebene Zusammensetzung bzw. das Präparat wird zur Prophylaxe von Schnupfen eingesetzt.

Weiterhin können bei Erkältungskrankheiten bakterielle Infektionen, die sich auf die bereits bestehende Virusinfektion "aufsetzen", auftreten. Derartige Infektionen werden als bakterielle Sekundärinfektionen oder bakterielle Superinfektionen bezeichnet. Die erfindunsgemäße Verwendung der Zusammensetzung betrifft auch die Prophylaxe dieser bakteriellen Sekundärinfektionen.

Erfindungsgemäß wird die Pflanze Cistus zur Herstellung einer Zusammensetzung für die Prophylaxe von grippalen Infekten verwendet. Von der Gattung Cistus sind 20 Arten bekannt:
C. *albidus* L.
C. *chinamadensis* Banares & P. Romero
C. *clusii* Dunal
C. *crispus* L.
C. *heterophyllus* Desf.
C. *incanus* (auch als C. *creticus* bezeichnet)
C. *inflatus* Pourr. Ex Demoly (auch als C. *hirsutus* Lam. oder C. *psilosepalus* Sweet bezeichnet)
*C. ladanifer* L*.*
C. *laurifolius* L.
C. *libanotis* L.
C. *monspeliensis* L.
C. *munbyl* Pomel
C. *ochreatus* Chr. Sm. ex Buch
C. *osbeckiifolius* Webb ex Christ.
C. *parviflorus* Lam.
C. *populifolius* L.
C. *pouzolzii* Delile
C. *salviifolius* L.
C. *sintenisii* Litard*.* (auch als C. *albanicus* E.F. Warburg ex Heywood bezeichnet)
C. *symphytifolius* Lam.

Bevorzugt wird die Zusammensetzung aus der Art C. *incanus* gewonnen. C. *incanus* umfasst zwei Unterarten, C. *incanus* ssp. tauricus sowie C. *incanus* ssp. undulatus. Von diesen wird besonders bevorzugt die Unterart C. *incanus ssp.* tauricus für die Zusammensetzung verwendet.

Die einzelnen Inhaltsstoffe der Cistus-Pflanze sind noch nicht vollständig bekannt. Es wurde jedoch bereits nachgewiesen, dass Cistus einen hohen Gehalt an Polyphenolen aufweist. Von den Polyphenolen sind insbesondere die Flavonoide stark vertreten.

Flavonoide bestehen grundsätzlich aus zwei aromatischen und einem sauerstoffhaltigen heterocyclischen Ring. Anhand von strukturellen Unterschieden am O-heterocyclischen Ring lassen sich die Flavonoide in die folgenden sechs Gruppen einteilen: Flavonole, Flavanole, Flavanone, Flavone, Anthocyane und Isoflavanoide. In Cistus-Pflanzen nachgewiesen wurden unter anderem Flavonole, wie Gluercetin und Myricetin und deren Glykoside, Flavan-3-ole (Catechine), wie (+)-Gallocatechin und (+)-Gallocatechin-3-O-gallat, sowie Di- und Oligomere der Catechine, die Proanthocyanidinen.

Weitere Inhaltsstoffe sind α-Pinen, Camphen und Terpenoide, wie Labda-7,13-dien-15-ol, 8α, 15-Labdandiol, Labdanolsäure, Laurifolinsäure, Acetyllaurifolinsäure, 8,13-Epoxy-15-dimethoxy-ent-labdan, 3α-Hydroy-ent-13-epimanoyloxid (=Ribenol), Salmantsäure, Salmantidiol, Halimsäure, Dihydrohalimsäure, 2β-Hy-droxy-dihydrohalimsäure, Cistodisäure, Cistodiol, ent-Kauran-16,17-diol, dihydroabietinsäuremethylester, Cabraleon und 20,25-Epoxy-24-hydroxydammaran-3-on [R. Hegnauer, Chemotaxonomie der Pflanzen Bd. VIII S. 1989, 246-247].

Die erfindungsgemäß verwendete Zusammensetzung wird aus den oberirdischen Teilen der Pflanzen gewonnen. Bevorzugt werden die im selben Jahr nachgewachsenen oberirdischen Triebe der Pflanzen verwendet. Es können alle Bestandteile des oberirdischen Teils der Pflanze, wie Blätter, Stengel oder Blüten, verwendet werden. Bevorzugt werden die Stengel mit Blättern und Blüten eingesetzt. Die Pflanzenteile können direkt nach der Ernte, also in rohem Zustand, gegebenenfalls in zerkleinerter Form, entweder getrocknet oder ausgepresst werden, um einen Presssaft herzustellen. In einer weiteren Ausführungsform werden die Pflanzenteile in rohem Zustand einer Extraktion mit einem Lösungsmittel, wie beispielsweise einer Mazeration oder Perkolation, unterworfen. Alternativ können die Pflanzenteile vor der Extraktion auch getrocknet und/oder anschließend in geeigneter Weise zerkleinert werden, indem sie beispielsweise gerieben oder geschnitten werden.

Für die erfindungsgemäß verwendete Zusammensetzung werden getrocknete und gegebenenfalls zerkleinerte Pflanzenbestandteile, der ausgepresste Pflanzensaft oder ein Extrakt verwendet. Erfindungsgemäß wird der Begriff "Extrakt" stellvertretend für alle Produkte verwendet, die durch eine Extraktion mit einem Lösungsmittel, wie einer Mazeration oder Perkolation, erhalten werden.

Bevorzugt liegt die Zusammensetzung in Form eines Extraktes aus der Cistus-Pflanze vor.

Allgemein erfolgt eine Extraktion mit einem geeigneten Lösungsmittel. Geeignete Lösungsmittel sind Wasser, Alkohole, wie Methanol, Ethanol oder Isopropanol, oder chlorierte Lösungsmittel, wie Dichlormethan, sowie Aceton, Acetylaceton, Ethylacetat, Ammoniak oder Eisessig, aber auch superkritisches Kohlendioxid. Es können auch Mischungen der genannten Lösungsmittel eingesetzt werden. Bevorzugt wird Wasser oder ein Gemisch aus Wasser mit Methanol oder Ethanol eingesetzt.

Die Extraktion wird üblicherweise bei Temperaturen von 25°C bis gegebenenfalls zum Siedepunkt des eingesetzten Lösungsmittel durchzuführen. Bevorzugt ist eine Extraktion bei 95 bis 100°C.

Weiterhin können auch Fette, wie Schweineschmalz, Wachse, wie Bienenwachs, oder Öle, wie Olivenöl und Mandelöl, zur Extraktion verwendet werden. Bevorzugt wird Mandelöl eingesetzt.

Um eine möglichst hohe Ausbeute zu erreichen, kann das Pflanzenmaterial mehrfach extrahiert werden. Hierbei können in den verschiedenen Extraktionsschritten auch unterschiedliche Lösungsmittel zum Einsatz kommen oder einer Extraktion mit einem Lösungsmittel kann eine Extraktion mit einem Fett, Wachs oder Öl folgen und umgekehrt.

Durch die Extraktion wird ein flüssiges, halbfestes oder festes Rohprodukt erhalten, das in dieser Form zur Herstellung einer Zusammensetzung zur Prophylaxe und/oder Behandlung von Erkältungskrankheiten eingesetzt werden kann.

Eine Mazeration wird üblicherweise für fünf bis neun Tage, bevorzugt für sieben Tage, mit einem Gemisch aus Wasser und Ethanol bei Raumtemperatur durchgeführt, indem die Pflanzenbestandteile mit dem Lösungsmittelgemisch übergossen und für den genannten Zeitraum stehen gelassen werden.

Eine Perkolation der Pflanzenteile wird erfindungsgemäß üblicherweise durch Behandeln der Teile mit Wasser bei 95 bis 100°C für vier bis fünf Stunden erreicht, indem das Wasser durch die Pflanzenteile geleitet wird.

Das aus einer Extraktion mit einem Lösungsmittel, wie einer Mazeration oder einer Perkolation, erhaltene Rohprodukt kann vor der Verwendung auch aufkonzentriert und/oder getrocknet und/oder weiter aufgearbeitet werden. Die Aufarbeitung kann beispielsweise dem Fachmann geläufige Reinigungsschritte, wie Zentrifugation, Filtration und Dekantieren, einschließen, um suspendierte Stoffe aus dem Extrakt zu entfernen.

Ein so erhaltener Extrakt kann schließlich weiter zu einem Trockenextrakt verarbeitet werden. Zur Herstellung des Trockenextrakts kann dem flüssigen Rohextrakt, dem aufkonzentrierten Extrakt oder dem gereinigten Extrakt das Lösungsmittel beispielsweise durch Sprühtrocknung, Gefriertrocknung oder Vakuumtrocknung entzogen werden.

Die Zusammensetzung wird zur Prophylaxe von Erkältungskrankheiten eingesetzt, die durch Rhinovirenhervorgerufen werden.

Für die medizinische Verwendung kann die Zusammensetzung in jeder dem Fachmann geläufigen Applikationsform angewendet werden, z.B. als Tabletten, Filmtabletten, Brausetabletten, Kapseln, Pulver, Granulate, Dragees, Salben, Cremes, Gele, Lösungen oder Sprays.

In galenischen und anderen Applikationsformen kann die Zusammensetzung hierbei mit den üblichen galenischen Hilfsmitteln, wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien, Konsistenzgeber, Penetrationsverbesserer und/oder Treibgasen, verarbeitet werden.

Der erfindungsgemäß verwendeten Zusammensetzung können weitere Bestandteile, wie Vitamine und Mineralstoffe, beigesetzt werden.

Die Zusammensetzung kann beispielsweise auch Futtermitteln oder Lebensmitteln, wie Getränken, beigesetzt werden. In Form eines Extrakt kann die Zusammensetzung auch selbst als Tee aufgegossen werden. Es können jedoch auch direkt die Pflanzenteile, beispielsweise die Blätter der Cistus-Pflanzen für die Teezubereitung mit heißem Wasser übergossen werden. Weiterhin kann die Zusammensetzung Bestandteil von Nahrungsergänzungsmitteln sein, deren Einnahme in den Wintermonaten dazu beitragen kann, die Abwehrkräfte zu stärken und somit beispielsweise einer Schnupfeninfektion vorzubeugen.

In einen weiteren Ausführungsform kann die Zusammensetzung erfindungsgemäß als Lösung, insbesondere als Gurgellösung, zur Prophylaxe von Erkältungskrankheiten, insbesondere von Entzündungen im Mund- und Rachenraum, verwendet werden.

Die Zusammensetzung kann auch gemischt mit Bestandteilen aus weiteren Pflanzen, wobei die Bestandteile bevorzugt in Form von Pflanzenextrakten vorliegen, verwendet werden. Bevorzugt werden Bestandteile von Pflanzen oder Pflanzenextrakte von gleichartiger oder synergistischer Wirkung verwendet.

Je nach Art der Applikation variiert die Konzentration der Zusammensetzung in der Anwendungsform. In der Regel beträgt die Menge der Zusammensetzung zwischen 0,5 bis 1000 mg pro Dosierungseinheit bei festen Applikationsformen. Bevorzugt beträgt die Menge der Zusammensetzung zwischen 1 bis 500 mg pro Einheit. In flüssigen Applikationsformen kann die Zusammensetzung in einer Konzentration von 1 µg/ml bis 100 mg/ml vorhanden sein, bevorzugt von 25 µg/ml bis 50 mg/ml. Bei halbfesten Applikationsformen beträgt der Gehalt an Zusammensetzung 1 bis 90 Gew.-%, bevorzugt 5 bis 75 Gew.-%.

Bevorzugt wird die Zusammensetzung in Form einer Tablette verabreicht. Es ist bevorzugt, dass die Zusammensetzung hierbei als Extrakt vorliegt. Ganz besonders bevorzugt liegt die Zusammensetzung als Trokkenextrakt vor.

Es ist weiterhin bevorzugt, die Zusammensetzung in Form von Emulsionen, Salben, Gelen oder Cremes zur topischen Applikation zu verabreichen. Hierbei wird die Zusammensetzung vorzugsweise in Form eines Extrakts eingesetzt, in welchem die Wirkstoffe durch Extraktion mit einem Fett, Wachs oder Öl der Pflanze entzogen worden sind. Es ist außerdem bevorzugt, dass dieser Extrakt zu einem Trockenextrakt weiter verarbeitet wird, der anschließend mit einem Fett, Wachs oder Öl vermischt oder in diesen gelöst wird.

Außerdem ist es bevorzugt, dass die Zusammensetzung als Aerosol oder als Raumspray vorliegt. Vorzugsweise wird hierzu ein flüssigeroderfester Extrakt von Cistus verwendet. Neben dem Extrakt kann das Aerosol oder das Raumspray pharmazeutisch unbedenkliche Substanzen, Träger und Hilfsstoffe enthalten. Das Aerosol oder das Raumspray kann zur Desinfektion von Gegenständen und Räumlichkeiten verwendet werden, mit denen Erkältungsviren in Kontakt gekommen sind oder potentiell in Kontakt treten könnten, insbesondere Verkehrsmitteln jeder Art, in denen Menschen, Tiere und/ oder Lebensmittel transportiert werden. Beispielsweise kann ein Flugzeug vor dem Start mit dem erfindungsgemäßen Aerosol oder dem erfindungsgemäßen Raumspray ausgesprüht werden, um eine Verbreitung der Erkältungsviren zu unterbinden und somit die Anstekkungsgefahr für den Menschen zu minimieren. Das Aerosol oder das Raumspray kann auch in Gegenwart von Menschen, z.B. in Warteräumen, versprüht werden, da es keinerlei toxische Wirkungen beim Menschen hervorruft.

Weiterhin kann die Zusammensetzung auch als Nasenmittel oder als Inhalationslösung verabreicht werden. Das Nasenmittel kann als Nasenspray oder als Nasengel zum Einsatz kommen. Zur Verabreichung können verschiedene Applikatoren und Dispersionssysteme zum Einsatz kommen.

Die erfindungsgemäße Verwendung von Cistus ist nicht auf den Menschen beschränkt, sondern kann auch bei Tieren, insbesondere Säugetieren, wie Haustieren oder Nutztieren, eingesetzt werden.

Das folgende Beispiel erläutert die vorliegende Erfindung.

Ein Cistus-Extrakt wurde hinsichtlich seiner Zelltoxizität und -viabilität sowie auf seine antivirale Aktivität gegenüber Rhinoviren getestet. Zu diesem Zweck wurde der Extrakt in PBS (steril) unter Erhitzen (1 h/ 100°C) gelöst (Stock-Lösung 1 mg/ml). Die Dosierung für die in vitro Studien betrug 100 µg/ml System.
Als Virusisolate diente humanes Rhinovirus Typ 14.
Als Wirtszelllinien dienten HeLa-Zellen (humane Cervix-Karzinomzelllinie).

Zur Bestimmung der Eigenschaften des Extraktes wurden die folgenden Untersuchungsmethoden herangezogen.

### Mikroskopische Untersuchungen:

Bei den mikroskopische Untersuchungen wurden A549 Lungenepithelzellen und MDCK (Madin-Darby canin kidney cells) Hundenierenepithelzellen für verschiedene Zeitpunkte (9h, 24h, 32h, 48h) mit verschiedenen Konzentrationen des Extrakts (2, 10, 25, 50 µg/ml) behandelt und danach lichtmikroskopisch untersucht. Die Experimente wurden zweifach kontrolliert durchgeführt.

### Viabilitätstests:

Bei den Viabilitätstests wurden A549 Lungenepithelzellen und MDCK Hundenierenepithelzellen für verschiedene Zeitpunkte (24h, 48h, 56h, 72h) mit verschiedenen Konzentrationen des Extrakt (2, 10, 25, 50 µg/ml) behandelt und danach mit Propidiumiodid angefärbt, um das Verhältnis toter und lebender Zellen durchflußzytometrisch zu bestimmen. Die Experimente wurden insgesamt viermal durchgeführt.

### Untersuchung der apoptotischen Caspaseaktivierung:

Zur Untersuchung der apoptotischen Caspaseaktivierung wurden A549 Zellen für 48h mit 25 und 50 µg/ml des Extraktes behandelt. Die Zellen wurden darauf hin lysiert, die zellulären Proteine gelelektrophoretisch aufgetrennt und im Western Blot mit einen anti-PARP Antikörper (Poly(ADP-Ribose)Polymerase, Caspase Substrat) auf die apoptotische Spaltung dieses Proteins durch Caspasen hin untersucht. Als positiver Kontrollstimulus diente der Apoptose-Induktor Staurosporin. Die Experimente wurden in zwei parallelen Ansätzen durchgeführt.

### Beispiel

### Herstellung eines Extraktes aus Cistus incanus ssp. tauricus

Zur Extraktion werden die nachgewachsenen oberirdischen Triebe (Blätter, Blüten und Stengel) verwendet. Das Pflanzenmaterial wird bei Raumtemperatur im Schatten bis zu einem Restwassergehalt von maximal 10% im Freien getrocknet. Anschließend werden die Pflanzenteile auf eine Größe von ≤ 8 mm geschnitten.

Die geschnittenen Pflanzenteile werden einer Perkolation bei 95 bis 100°C mit der zehnfachen Menge an gereinigtem Wasser Ph.Eur. für 4 bis 5 Stunden unterworfen. Die gewonnene Lösung wird mittels eines Plattenverdampfers bei einer Dampftemperatur von 75 bis 80°C auf 18 bis 19% des ursprünglichen Volumens aufkonzentriert. Der Gehalt an Trockensubstanz beträgt ca. 45%.

Mittels eines Rührwerksverdampfers wird der Gehalt an Trockensubstanz auf 50 bis 51 % erhöht, indem der Extrakt für vier Stunden bei 110 bis 114°C unter reduziertem Druck (0,6 bar) erhitzt wird. Anschließend wird der Extrakt für 1 Stunde bei 100,3°C aufgekocht, um einen Gehalt an Trockensubstanz von ca. 53% zu erhalten.

Schließlich wird eine Vakuumband-Trocknung bei 16 mbar mit absteigendem Temperaturgradienten (140°C, 120°C, 90°C, 20°C) durchgeführt. Der Gehalt an Trockensubstanz beträgt 92 bis 93%. Abschließend wird der Extrakt vermahlen. Aus diesem Extrakt wird dann die vorstehend beschriebene Stock-Lösung hergestellt.

### Mikroskopische Untersuchungen

Bei den Mikroskopaufnahmen der Untersuchungsreihen mit MDCK Zellen und A549 Zellen konnten bei keiner der verwendeten Konzentrationen des Extrakts und der untersuchten Zeitwerte signifikanten Veränderungen hinsichtlich Zellzahl und Zellmorphologie im Vergleich zu den unbehandelten Kontrollproben festgestellt werden.

### Viabilitätstest

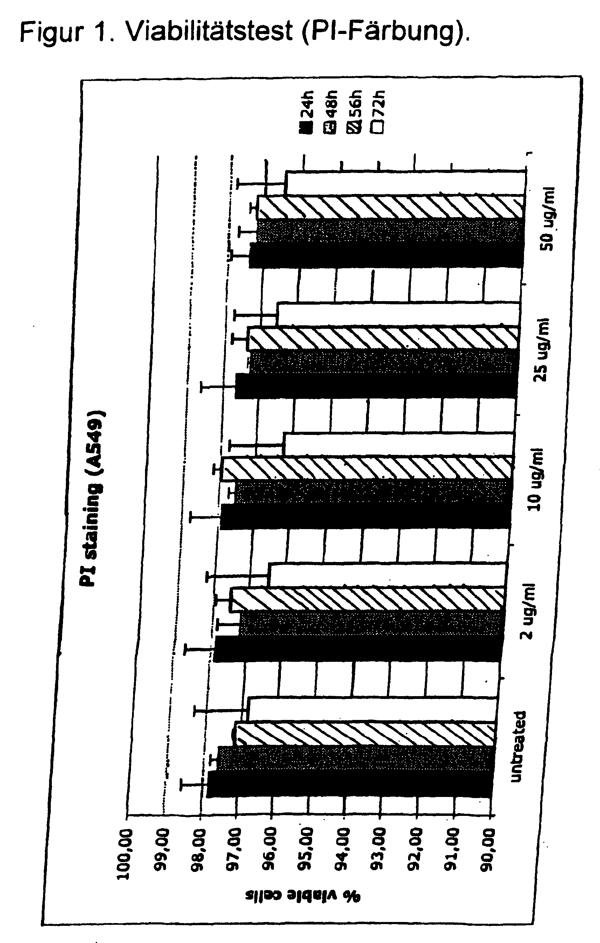

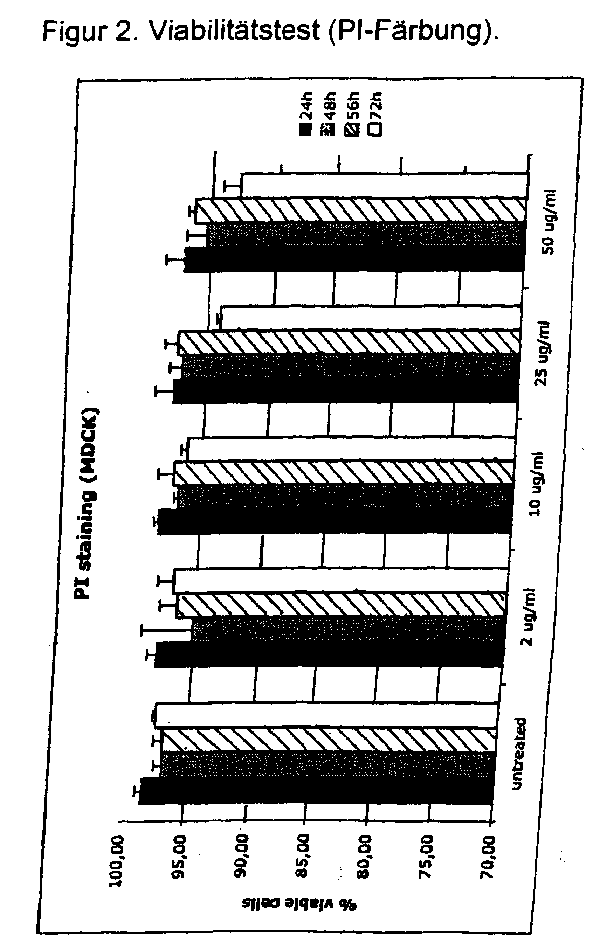

Resultate der Viabilitätstest sind in Fig. 1 und 2 gezeigt, in denen vergleichend die Anzahl der lebenden Zellen aus den jeweiligen Proben im Mittelwert aus den vier durchgeführten Bestimmungen zusammenfassend dargestellt sind. Im Ergebnis war im gesamten Beobachtungszeitraum von 72 Stunden kein negativer Einfluss des Extraktes auf das Überleben von MDCK oder A549 Zellen festzustellen.

### Untersuchung der apoptotischen Caspaseaktivierung

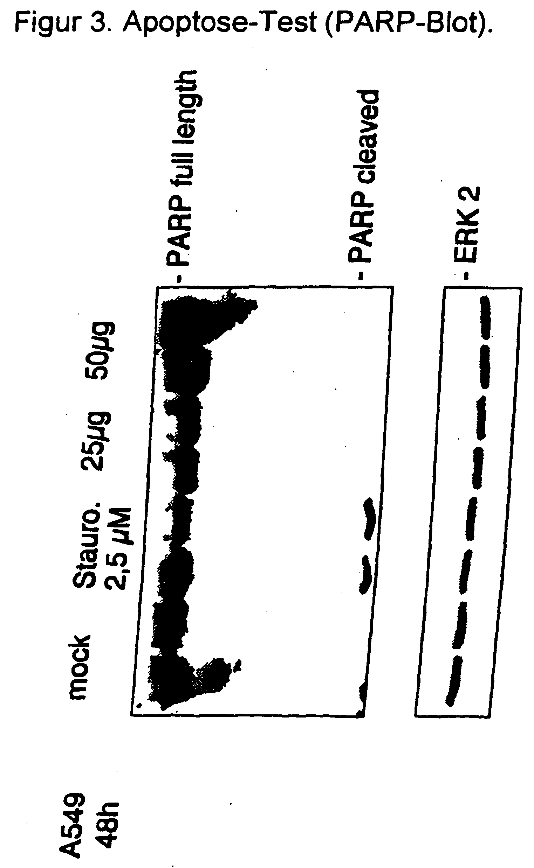

Ergebnisse aus der Untersuchung der apoptotischen Caspaseaktivierung sind in Fig. 3 gezeigt, in der Ergebnisse der Westem Blot Analyse zur Bestimmung der Caspaseaktivität dargestellt sind. Während der Kontrolistimulus Staurosporin (Stauro) zu einer effizienten Spaltung des Caspase Substrats Poly(ADP-Ribose)Polymerase führt (Bande PARP cleaved), ist eine solche Aktivität weder in unbehandelten (mock) noch in extraktbehandelten Zellen (25 µg/ml, 50 µg/ml) nachzuweisen. Ein Kontrollblot gegen das Protein ERK2 diente als Kontrolle für einheitliche Proteinbeladung. Im Ergebnis ist zu folgern, dass Behandlung mit Pflanzenextrakt in den verwendeten Konzentrationen und im Beobachtungszeitraum nicht zur Caspaseaktivierung und Apoptoseinduktion führt.

Die Untersuchungen zur Morphologie, Viabilität und Caspase Aktivierung der mit Cistus-Extrakt behandelten Zellen zeigen, dass Konzentration bis zu 50 µg/ml des Extraktes keine signifikant toxische Wirkung auf die hier genutzten Wirtszellen A549 und MDCK ausweisen und weder vermehrt nekrotischen noch apoptotischen Zelltod induzieren. Darüber hinaus konnte keine signifikante Reduktion der Zellzahlen erkannt werden, so dass auch das Zellwachstum durch die Extraktwirkung nicht eingeschränkt ist.

### Untersuchungen zur antiviralen Aktivität:

### Rhinoviren Anzucht:

Vier T175 Flaschen mit 80% konfluenten HeLa-Zellen wurden mit humanen Rhinovirus Typ 14 (HRV) angeimpft und für eine Woche bei 33°C inkubiert. Dafür werden 50µl HRV14 (Virustiter 10⁸/ml TCID (Tissue culture infectious dose)) in 16 ml Infektionsmedium (D-MEM (Dulbecco's Modified Eagle Medium), 2% FCS (Fetal Calf Serum), 10-20 mM MgCl₂) gemischt und 4 ml je T175 gegeben. Jede T175 wird noch mit 10 ml Infektionsmedium aufgefüllt, so dass in jeder T175 14 ml Infektionsmedium sind. Sobald sich 70% der adhärenten Zellen lösen, wird das Virus geerntet.

### Aufreinigung der Rhinoviren:

Der Virusüberstand wird zunächst bei 3000 rpm für 30 min zentrifugiert, um das Zellpellet zu entfernen. In der Ultrazentrifuge wird der Virusüberstand bei 35000 rpm zentrifugiert (Rotor Typ SW41 Ti in Beckman Polyallomer Röhrchen) für drei Stunden bei 4°C auf Succrose-Kissen (1,5 ml Succrose 65% in Wasser, 300 µl 10xPBS (Phosphatebuffered saline), 1,2 ml Wasser) und das Pellet in 100 µl Infektionsmedium aufgenommen. Weitere Virusaufkonzentrierung erfolgt mit einem 100 kDa Ausschlussfilter (Centricon YM100) bei 3300 rpm für eine Stunde bei 4°C. Das Retentat enthält das aufgereinigte Viruskonzentrat, das Filtrat wird entsorgt.

### Untersuchung der antiviralen Aktivität (Tissue culture infectious dose (TCID) Assay:

Am Vortag werden 5 x 104 HeLa-Zellen in D-MEM Medium pro 96-Well Platte ausgesät, so dass die Zellen am nächsten Tag ca. 70% konfluent sind. Das D-MEM Medium wird am nächsten Tag abgesaugt und durch 90 µl Infektionsmedium (D-MEM, 2% FCS, 10-20 mM MgCl₂) ersetzt. Die Infektion erfolgt in der höchsten Konzentration von 100 µg/ml mit 10 µl der virushaltigen Lösung in der ersten 96-Well Reihe. Nachdem diese 100 µl durch auf und ab pipettieren gemischt wurden, werden davon 10 µl jeweils in die zweite 96-Well Reihe gegeben. Der Vorgang wird mit den weiteren Reihen wiederholt (1: 10 Verdünnungsreihe). Es werden parallel zwei oder drei Ansätze gefahren, die zur Auswertung herangezogen werden.

Die so bearbeitete Platte wird für fünf Tage bei 33°C im Brutschrank inkubiert. Am fünften Tag wird die Platte mindestens zweimal mit PBS gewaschen und mit 100 µl pro 96-Well Kristallviolett (0,07% in purem Ethanol) für fünf Stunden gefärbt. Die Platte wird anschließend mehrmals in Wasser gewaschen, ausgeklopft (ca. 10mal) und getrocknet. Eine Blaufärbung zeigt lebende Zellen an. Tote Zellen wurden aus den Wells gewaschen und hinterlassen einen weißen Hintergrund.

Um die Wirkung von Cistus-Extrakt auf die Infektiosität von HRV14 zu testen, wurde entweder das Infektionsmedium mit 100 µg/ml Cistus-Extrakt versetzt oder es wurden zusätzlich die Viren eine Stunde lang mit 100 µg/ml Cistus-Extrakt vorbehandelt.
Im Ergebnis zeigte sich, dass der Cistus-Extrakt sowohl im Infektionsmedium als auch nach zusätzlicher Vorinkubation der Viren in allen durchgeführten Ansätzen in der Lage war, die Infektion und damit die Zerstörung des Zellrasens zu verhindern. Der Cistus-Extrakt in der verwendeten Konzentration hatte dabei keinerlei erkennbare schädigende Einflüsse auf die Zellen. Damit ist eine hemmende Wirkung des Cistus-Extrakte auf Infektiosität von Rhinoviren bewiesen.

## Patentansprüche

1. Verwendung von Cistus zur Herstellung einer Zusammensetzung mit antiviraler Aktivität gegen Rhinoviren zur Hemmung der Infektiosität von Rhinoviren bei der Prophylaxe von Erkältungskrankheiten, wobei die Erkältungskrankheit eine Primärinfektion, hervorgerufen durch Rhinoviren, umfasst.

2. Verwendung nach Anspruch 1, worin die Pflanze aus Cistus *incanus* ausgewählt wird.

3. Verwendung nach Anspruch 1 oder 2, worin die oberirdischen Teile der Pflanze eingesetzt werden. 3

4. Verwendung nach mindestens einem der Ansprüche 1 bis 3, worin die Zusammensetzug in flüssiger, trockener oder halbfester Form vorliegt.

5. Verwendung nach mindestens einem der Ansprüche 1 bis 4, worin die Zusammensetzung in Form eines Extraktes eingesetzt wird.

6. Verwendung nach Anspruch 5, worin der Extrakt ein wässriger Extrakt oder ein alkoholischer Extrakt ist.

7. Verwendung nach mindestens einem der Ansprüche 1 bis 6, wobei die Zusammensetzung oral oder topisch verabreicht wird.

8. Verwendung nach mindestens einem der Ansprüche 1 bis 7, wobei die Zusammensetzung als Nasenmittel oder Inhalationsmischung vorliegt.

9. Verwendung nach mindestens einem der Ansprüche 1 bis 7, wobei die Zusammensetzung als Aerosol oder Raumspray vorliegt.

10. Verwendung nach mindestens einem der Ansprüche 1 bis 5 und 7, wobei die Zusammensetzung in Form einer Tablette, einer Filmtablette, einer Brausetablette, einer Kapsel, eines Pulvers, eines Granulats oder eines Dragees vorliegt.

11. Verwendung nach mindestens einem der Ansprüche 1 bis 7, wobei die Zusammensetzung in Form einer Salbe, eines Gels oder einer Creme vorliegt.

12. Verwendung nach mindestens einem der Ansprüche 1 bis 7, wobei die Zusammensetzung als Gurgellösung oder als Pflanzensaft vorliegt.

## Claims

1. Use of Cistus for producing a composition having antiviral activity against rhinoviruses for inhibiting the infectivity of rhinoviruses in the prophylaxis of common cold diseases, wherein the common cold disease comprises a primary infection, caused by rhinoviruses.

2. Use according to Claim 1, wherein the plant is chosen from Cistus *incanus.*

3. Use according to Claim 1 or 2, wherein the aboveground parts of the plant are used.

4. Use according to at least one of the Claims 1 to 3, wherein the composition is in liquid, dry or semi-solid form.

5. Use according to at least one of the Claims 1 to 4, wherein the composition is used in the form of an extract.

6. Use according to Claim 5, wherein the extract is an aqueous extract or an alcoholic extract.

7. Use according to at least one of the Claims 1 to 6, wherein the composition is administered orally or topically.

8. Use according to at least one of the Claims 1 to 7, wherein the composition is present as a nasal agent or inhalation mixture.

9. Use according to at least one of the Claims 1 to 7, wherein the composition is present as an aerosol or room spray.

10. Use according to at least one of the Claims 1 to 5 and 7, wherein the composition is present in the form of a tablet, coated tablet, effervescent tablet, capsule, powder, granulate or sugar-coated tablet.

11. Use according to at least one of the Claims 1 to 7, wherein the composition is present in the form of an ointment, gel or cream.

12. Use according to at least one of the Claims 1 to 7, wherein the composition is present as a gargling solution or plant juice.

## Revendications

1. Utilisation de Cistus pour la preparation d'une composition ayant une activité antivirale contre les rhinovirus pour l'inhibition de l'infectivité des rhinovirus dans la prophylaxie de maladies de refroidissements, la maladie de refroidissement comprenant une infection primaire, provoquée par des rhinovirus.

2. Utilisation suivant la revendication 1, dans laquelle la plante est sélectionnée parmi les Cistus *Incanus.*

3. Utilisation suivant la revendication 1 ou 2, dans laquelle sont utiliseés les parties en surface de la plante.

4. Utilisation suivant l'une au moins des revendications 1 à 3, dans laquelle la composition est présente en forme liquide, sèche ou semi-solide.

5. Utilisation suivant l'une au moins des revendications 1 à 4, dans laquelle la composition est utilisée en forme d'un extrait.

6. Utilisation suivant la revendication 5, dans laquelle l'extrait est un extrait aqueux ou un extrait à base d'alcool.

7. Utilisation suivant l'une au moins des revendications 1 à 6, dans laquelle la composition est administrée par voie orale ou topique.

8. Utilisation suivant l'une au moins des revendications 1 à 7, dans laquelle la composition est présente sous forme d'agent nasal ou de mélange inhalateur.

9. Utilisation suivant l'une au moins des revendications 1 à 7, dans laquelle la composition est présente sous form d'aérosol ou de spray.

10. Utilisation suivant l'une au moins des revendications 1 à 5 et 7, dans laquelle la composition est présente en forme d'un comprimé, d'un comprimé pelliculé, d'un comprimé effervescent, d'une gélule, d'une poudre, d'un granulat ou d'une dragée.

11. Utilisation suivant l'une au moins des revendications 1 à 7, dans laquelle la composition est présente en forme d'une pommade, d'un gel ou d'une crème.

12. Utilisation suivant l'une au moins des revendications 1 à 7, dans laquelle la composition est présente sour forme de gargarisme ou de jus de plantes.
